# FASCICULE DE BREVET EUROPEEN

(11) **EP 3 380 016 B1**
(45) Date de publication et mention de la délivrance du brevet: **09.09.2020**
(21) Numéro de dépôt: 16800962.9
(22) Date de dépôt: 23.11.2016
(51) Int. Cl.: A61B 6/08, A61B 6/00

(54) **ENSEMBLE DE RADIOLOGIE ET PROCEDE D'ALIGNEMENT D'UN TEL ENSEMBLE**
RADIOLOGISCHE ANORDNUNG UND VERFAHREN ZUM AUSRICHTEN SOLCH EINER ANORDNUNG
RADIOLOGY ASSEMBLY AND METHOD FOR ALIGNING SUCH AN ASSEMBLY

(30) Priorité: 23.11.2015 FR 1561250
(43) Date de publication de la demande: 03.10.2018
(73) Titulaire: Trixell, 38430 Moirans (FR)
(72) Inventeur: MUNIER, Bernard, 38430 Moirans (FR); JOLY, Guillaume, 38430 Moirans (FR); VERMONT, Fabien, 38246 Meylan Cedex (FR); MENEROUD, Patrick, 38246 Meylan Cedex (FR); SOSNICKI, Olivier, 38246 Meylan Cedex (FR)
(74) Mandataire: Collet, Alain
(86) Numéro de dépôt international: PCT/EP2016/078563
(87) Numéro de publication internationale: WO 2017/089403

(56) Documents cités:
- EP-A1- 2 617 359
- WO-A2-2007/149402
- US-A1- 2012 230 473
- US-A1- 2014 376 700

## Description

L'invention concerne un ensemble de radiologie et plus précisément l'alignement de deux éléments de l'ensemble de radiologie, à savoir le capteur plan par rapport au tube générateur du faisceau de rayons X. L'invention concerne aussi un procédé d'alignement d'un tel ensemble de radiologie. L'invention se situe dans le domaine de la radiologie (par exemple médicale ou vétérinaire).

Dans le présent brevet, l'invention est présentée dans un cas d'application à un ensemble de radiologie. Néanmoins, l'invention peut trouver application dans d'autres domaines nécessitant un bon positionnement de deux éléments l'un par rapport à l'autre.

Un ensemble de radiologie est constitué de deux éléments : un tube générateur d'un faisceau de rayons X et un capteur plan d'images radiographiques. L'ensemble est destiné à réaliser principalement des images radiographiques de patients en milieu hospitalier. Un patient, dont on souhaite faire une radiographie, est placé entre le tube générateur d'un faisceau de rayons X et le capteur plan. Les deux éléments doivent donc être bien positionnés l'un par rapport à l'autre, de façon à ce que tous les rayons X émis par le tube générateur du faisceau de rayons X soient captés par le capteur plan. On parle alors de bon alignement entre les deux éléments. L'alignement doit se faire avant que les rayons X ne soient émis par le tube générateur du faisceau de rayons X. Le but est d'éviter de sur-irradier le patient avec des rayons X arrivant en dehors du capteur.

De manière générale, le tube générateur du faisceau de rayons X est aligné manuellement par un opérateur en face du capteur plan. L'alignement se fait en translation et en rotation. L'alignement se fait généralement lorsque le patient est en place, c'est-à-dire positionné entre le tube générateur du faisceau de rayons X et le capteur plan. Il existe de nombreux cas de figure où le capteur plan est masqué. On peut citer par exemple le cas où le capteur plan est placé sous un patient pour une radiographie de l'abdomen ou du bassin. On peut citer aussi le cas où le capteur plan est placé sous un drap, sous un brancard ou même dans une couveuse. Il est donc, dans ce cas, très difficile pour l'operateur d'effectuer l'alignement du tube générateur du faisceau de rayons X par rapport au capteur plan.

Par ailleurs, l'environnement du capteur plan peut être de plusieurs types. L'environnement peut notamment être un lit d'hôpital ou un brancard comportant des armatures métalliques ou une couveuse pour bébés prématurés. L'environnement du capteur peut donc constituer une gêne supplémentaire pour le bon positionnement du tube générateur par rapport au capteur plan.

L'alignement du premier élément par rapport au second élément comprend une correction de plusieurs défauts : défaut de centrage (le faisceau de rayons X n'est pas centré sur le capteur plan), défaut d'orientation (le faisceau de rayons X est mal orienté par rapport au plan du capteur plan) et défaut de perpendicularité (le faisceau de rayons X n'arrive pas perpendiculairement sur le capteur plan). Le défaut de perpendicularité est critique quand une grille anti-diffusante est utilisée pour réaliser l'image. La grille est alors placée sur le capteur plan. Les rayons X, pour pouvoir être détectés par le capteur plan, doivent arriver sur le capteur perpendiculairement au capteur plan. La tolérance angulaire par rapport à la perpendicularité est faible (seulement quelques degrés).

Il existe plusieurs façons de procéder à l'alignement de deux éléments. On peut d'abord citer un alignement optique où les deux éléments sont alignés au moyen de faisceaux de lumière qui mesurent la position relative d'un élément par rapport à l'autre. L'alignement optique ne peut pas s'appliquer dans le domaine de la radiologie puisque le capteur plan est souvent en partie masqué par un drap ou par le patient.

L'alignement peut aussi se faire au moyen de faisceaux d'ondes acoustiques. Or, comme l'alignement se fait en présence du patient, le patient peut masquer tout ou partie du capteur plan. De plus, la présence du patient peut atténuer localement les ondes acoustiques et ainsi fausser la mesure de distance entre le capteur plan et le tube générateur du faisceau de rayons X.

Il est aussi possible d'effectuer l'alignement de deux éléments en se basant sur la mesure de la durée de propagation d'une onde électromagnétique. La mesure de la durée de propagation d'une onde permet de mesurer la distance entre les deux éléments. Par triangulation, il est possible de repérer la position relative des deux éléments l'un par rapport à l'autre. Mais cet alignement ne s'applique pas avec succès dans le cas d'application à la radiologie puisque selon le positionnement du patient entre les deux éléments (tube générateur du faisceau de rayons X et capteur plan), la durée de propagation de l'onde électromagnétique peut varier. De plus, des échos multiples peuvent être générés à cause de l'environnement (lit, brancard, ...), les échos pouvant avoir des niveaux de signal supérieurs au signal principal.

Sur le même principe, il existe un alignement basé sur la mesure de l'atténuation d'un signal électromagnétique pour mesurer la distance entre deux éléments. Comme, dans le cas d'application à la radiologie, le patient peut atténuer localement l'onde électromagnétique et donc fausser la mesure, cet alignement n'est pas adapté.

Enfin, un système de radiologie dentaire (brevet FR 2 899 349) utilise plusieurs émetteurs de champ électromagnétique placés dans un même plan et un ou deux récepteurs de champ électromagnétique aptes à recevoir les champs électromagnétiques émis par les émetteurs. L'utilisation de deux récepteurs permet de déterminer l'orientation angulaire du capteur mais ne donne aucune précision sur l'angle d'un élément par rapport à l'autre (tube générateur par rapport au capteur plan). De plus, le positionnement des émetteurs dans un même plan donne une précision médiocre sur l'emplacement du capteur plan par rapport au tube générateur. Il est à préciser que la radiologie dentaire couvre une distance entre le tube générateur et le capteur relativement courte (20 à 30 cm) comparée à la distance entre le tube générateur et le capteur dans le domaine de la radiologie médicale (plutôt de l'ordre de 1 à 2 m).

L'invention vise à pallier tout ou partie des problèmes cités plus haut en proposant un ensemble de radiologie avec plusieurs émetteurs de champ électromagnétique, solidaires du tube générateur du faisceau de rayons X, qui sont positionnés dans des plans distincts et plusieurs capteurs de champ électromagnétiques positionnés sur le capteur plan recevant les rayons X. Cet ensemble permet de connaître sans ambiguïté la position du capteur plan dans l'espace et donc de connaître sa position par rapport au tube générateur du faisceau de rayons X.

A cet effet, le brevet a pour objet un ensemble de radiologie comprenant :
- un tube générateur d'un faisceau de rayons X centré autour d'une direction principale d'émission,
- un capteur plan sensiblement perpendiculaire à la direction principale d'émission, destiné à recevoir les rayons X,
caractérisé en ce qu'il comprend :
- un premier émetteur interrompu en deux parties d'émetteur de champ électromagnétique, disposé de façon à émettre un premier champ électromagnétique selon une direction principale sensiblement perpendiculaire à la direction principale d'émission, chacune des deux parties d'émetteur de l'émetteur interrompu étant positionnée de part et d'autre du faisceau de rayons X,
- des capteurs de champ électromagnétique solidaires du capteur plan, aptes à détecter les champs électromagnétiques émis par le premier émetteur et générer un signal électrique en fonction du champ électromagnétique détecté,
- un moyen de traitement du signal électrique destiné à déterminer la position relative du capteur plan par rapport au tube générateur en fonction du signal électrique.

Selon un mode de réalisation, l'ensemble de radiologie peut comprendre en outre un émetteur dit plan de champ électromagnétique, l'émetteur dit plan étant une bobine composée de spires, l'émetteur dit plan étant disposé de façon à émettre un champ électromagnétique selon une direction principale sensiblement parallèle à la direction principale d'émission, les spires étant traversées par la direction principale d'émission.

Selon un autre mode de réalisation, l'ensemble de radiologie peut comprendre un deuxième émetteur interrompu en deux parties d'émetteur de champ électromagnétique, disposé de façon à émettre un champ électromagnétique selon une direction principale sensiblement perpendiculaire à la direction principale d'émission et sécante à la direction principale du premier champ électromagnétique, chacune des deux parties d'émetteur de l'émetteur interrompu étant positionnée de part et d'autre du faisceau de rayons X. Les capteurs sont aptes à détecter le champ électromagnétique émis par le deuxième émetteur interrompu et générer un deuxième signal électrique en fonction du champ électromagnétique détecté.

Avantageusement, le moyen de traitement comprend des moyens pour distinguer les signaux électriques générés.

Selon un autre mode de réalisation, chacune des deux parties d'émetteur de l'émetteur interrompu comprend au moins une spire, la direction principale d'émission du faisceau de rayons X est positionnée entre les au moins une spire de l'émetteur interrompu.

Selon un autre mode de réalisation, l'émetteur dit plan comprend au moins une spire traversée par la direction principale d'émission du faisceau de rayons X.

Le brevet concerne également un procédé d'alignement d'un ensemble de radiologie selon l'invention comportant les étapes suivantes :
- Emission par l'émetteur du champ électromagnétique,
- Détection du champ électromagnétique par les capteurs,
- Génération d'un signal électrique par les capteurs en fonction du champ électromagnétique détecté,
- Détermination de la position relative du capteur plan par rapport au tube générateur par le moyen de traitement en fonction du signal électrique.

Selon un mode de réalisation, le procédé d'alignement selon l'invention comporte au préalable une étape de calibration destinée à étalonner le signal électrique en fonction de positions prédéterminées du tube générateur et du capteur plan.

Avantageusement, l'étape d'émission par le au moins un émetteur du au moins un champ électromagnétique comporte les étapes suivantes :
- Alimentation du premier émetteur interrompu par un premier signal électrique de façon à ce qu'il émette un premier champ électromagnétique dont la direction principale est sensiblement perpendiculaire à la direction principale d'émission,
- Alimentation du deuxième émetteur interrompu par un deuxième signal électrique de façon à ce qu'il émette un deuxième champ électromagnétique dont la direction principale est sensiblement perpendiculaire à la direction principale d'émission et sécante à la direction principale du premier champ électromagnétique.

Selon un mode de réalisation, l'étape d'émission par le au moins un émetteur du au moins un champ électromagnétique comporte l'étape suivante :
- Alimentation de l'émetteur dit plan par un troisième signal électrique de façon à ce qu'il émette un troisième champ électromagnétique dont la direction principale est sensiblement parallèle à la direction principale d'émission.

Avantageusement, l'alimentation des émetteurs se fait à des instants différents ou simultanément à des fréquences différentes ou simultanément en décalage de phase de façon à différencier les champs électromagnétiques émis.

L'invention sera mieux comprise et d'autres avantages apparaîtront à la lecture de la description détaillée d'un mode de réalisation donné à titre d'exemple, description illustrée par le dessin joint dans lequel :
- la figure 1 représente un mode de réalisation d'un ensemble de radiologie selon l'invention,
- la figure 2 représente un exemple de disposition des émetteurs de champ électromagnétique selon l'invention,
- la figure 3 représente un exemple d'un support des émetteurs de champ électromagnétique,
- la figure 4 représente une vue en coupe d'un ensemble de radiologie selon l'invention,
- la figure 5 représente schématiquement les étapes d'un procédé d'alignement selon l'invention.

Par souci de clarté, les mêmes éléments porteront les mêmes repères dans les différentes figures.

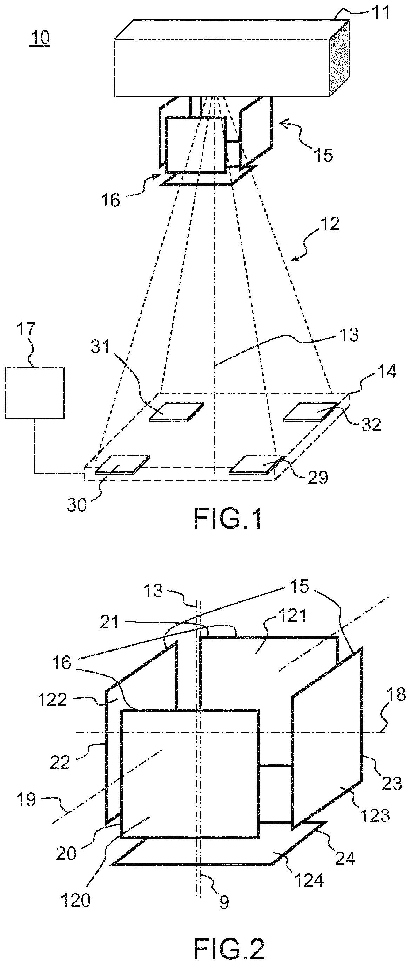

La figure 1 représente un mode de réalisation d'un ensemble de radiologie 10 selon l'invention. L'ensemble de radiologie 10 comprend un tube 11 générateur d'un faisceau de rayons X 12 centré autour d'une direction principale 13 d'émission. L'ensemble de radiologie 10 comprend un capteur plan 14 sensiblement perpendiculaire à la direction principale 13 d'émission. Le capteur plan 14 est destiné à recevoir les rayons X 12. Selon l'invention, l'ensemble de radiologie comprend un premier émetteur interrompu 15 en deux parties d'émetteur 20, 21 de champ électromagnétique disposé de façon à émettre un premier champ électromagnétique selon une direction principale sensiblement perpendiculaire à la direction principale 13 d'émission, chacune des deux parties d'émetteur 20, 21 de l'émetteur interrompu 15 étant positionnée de part et d'autre du faisceau de rayons X 12. Avantageusement, l'émetteur interrompu 15 est solidaire du tube 11 générateur du faisceau de rayons X 12. Dans cette configuration, la position du tube 11 générateur du faisceau de rayons X 12 peut se déduire de la direction principale du champ électromagnétique émis par l'émetteur interrompu 15.

De même, l'ensemble de radiologie peut comprendre un deuxième émetteur interrompu 16 en deux parties d'émetteur 22, 23 de champ électromagnétique, disposé de façon à émettre un champ électromagnétique selon une direction principale sensiblement perpendiculaire à la direction principale 13 d'émission et sécant à la direction principale du premier champ électromagnétique, chacune des deux parties d'émetteur 22, 23 de l'émetteur interrompu 16 étant positionnée de part et d'autre du faisceau de rayons X 12.

Autrement dit, chaque émetteur interrompu (par exemple 15) peut être considéré comme étant une paire d'émetteurs (20, 21) dont les faces principales sont parallèles entre elles, chacun des émetteurs étant situé de part et d'autre du faisceau de rayons X. La paire d'émetteurs 20, 21 (de même pour 22, 23) est équivalente à un émetteur virtuel qui serait situé entre les deux émetteurs 20, 21, dans le faisceau de rayons X. En considérant un émetteur interrompu (c'est-à-dire une paire d'émetteurs), le champ électromagnétique émis est équivalent au champ électromagnétique qui serait émis par l'émetteur virtuel équivalent. Cette disposition a l'avantage de ne pas obscurcir les rayons X puisque la paire d'émetteurs se trouve de part et d'autre du faisceau de rayons X et non pas dans leur faisceau. Par ailleurs, cette disposition des émetteurs a l'avantage de ne pas endommager les émetteurs. En effet, un émetteur équivalent placé dans le faisceau des rayons X serait endommagé par les rayons X au cours de son utilisation. Dans le cas de notre invention, les émetteurs ne sont pas soumis au rayonnement X et sont donc préservés du point de vue résistance des matériaux.

L'ensemble de radiologie 10 comprend aussi des capteurs 29, 30, 31, 32 de champ électromagnétique solidaires du capteur plan 14, aptes à détecter les champs électromagnétiques émis par les émetteurs 15, 16 et générer un signal électrique en fonction des champs électromagnétiques détectés. Généralement, chacun des capteurs de champ électromagnétique peut comprendre un circuit électronique d'amplification et de filtrage destiné à traiter le signal électrique généré par chacun des capteurs.

Enfin, l'ensemble de radiologie 10 comprend un moyen de traitement 17 du signal électrique destiné à déterminer la position relative du capteur plan par rapport au tube générateur 11 en fonction du signal électrique généré par les capteurs 29, 30, 31, 32.

La figure 2 représente un exemple de disposition des émetteurs 15 et 16 de champ électromagnétique selon l'invention. Sur la figure 2, l'ensemble de radiologie comprend deux émetteurs interrompus 15, 16 en deux parties d'émetteurs 20, 21 et 22, 23 de champ électromagnétique. Le premier émetteur interrompu 15 en deux parties d'émetteur 20, 21 de champ électromagnétique est disposé de façon à émettre un premier champ électromagnétique selon une direction principale 18 sensiblement perpendiculaire à la direction principale 13 d'émission. Chacune des deux parties d'émetteur 20, 21 de l'émetteur interrompu 15 est positionnée de part et d'autre du faisceau des rayons X 12. De même, le deuxième émetteur interrompu 16 en deux parties d'émetteur 22, 23 de champ électromagnétique est disposé de façon à émettre un premier champ électromagnétique selon une direction principale 19 sensiblement perpendiculaire à la direction principale 13 d'émission. Chacune des deux parties d'émetteur 22, 23 de l'émetteur interrompu 16 est positionnée de part et d'autre du faisceau des rayons X 12.

Un émetteur peut être par exemple une bobine ou un solénoïde. Un émetteur est constitué d'au moins une spire dans laquelle peut circuler un courant. Si on considère maintenant la surface représentée par la spire de chacune des parties d'émetteurs 20, 21 et 22, 23, on peut noter qu'une surface 120 de la partie d'émetteurs 20 est sensiblement parallèle à une surface 121 de la partie d'émetteurs 21. Et le champ électromagnétique émis par l'émetteur interrompu 15 a une direction principale 18 perpendiculaire aux surfaces 120 et 121. Sur le même principe, une surface 122 de la partie d'émetteurs 22 est sensiblement parallèle à une surface 123 de la partie d'émetteurs 23. Et le champ électromagnétique émis par l'émetteur interrompu 16 a une direction principale 19 perpendiculaire aux surfaces 122 et 123. Avantageusement, les surfaces 120 et 121 sont perpendiculaires aux surfaces 122 et 123. En plus d'être sécantes, les directions principales 18 et 19 sont alors sensiblement perpendiculaires entre elles. Cette disposition est notamment avantageuse si le tube générateur 11 du faisceau de rayons X 12 a un flux d'émission de forme carrée. Ainsi, le flux de rayons X 12 est émis selon la direction principale 13 d'émission, entre les surfaces 120, 121, 122, 123, sans intersecter les émetteurs 15, 16 (et donc sans les endommager), sans être obscurci puisque les émetteurs 15, 16 ne se situent pas dans le flux de rayons X 12. Autrement dit, chacune des deux parties d'émetteur 20, 21 ; 22, 23 de l'émetteur interrompu 15, 16 comprend au moins une spire et en ce que la direction principale 13 d'émission du faisceau de rayons X 12 est positionnée entre les au moins une spire de l'émetteur interrompu 15, 16.

En effet, cette disposition permet de faire en sorte que chacune des paires d'émetteurs 20, 21 et 22, 23 dont les surfaces respectives 120, 121 et 122, 123 sont parallèles entre elles (comme déjà dit la surface 121 est sensiblement parallèle à la surface 122, de même pour les surfaces 122 et 123) est équivalente à un émetteur virtuel situé au centre des surfaces 120, 121, 122, 123 des émetteurs 15, 16, au niveau de la direction principale 13 d'émission des rayons X, alors qu'il serait impossible de placer un émetteur unique au centre puisque le centre est occupé par le faisceau de rayons X. Ainsi les émetteurs peuvent émettre, en position décentrée, un champ électromagnétique équivalent à un champ électromagnétique émis en position centrée, sans obscurcir les rayons X émis par le tube générateur 11.

L'ensemble de radiologie 10 peut comprendre en outre un émetteur dit plan 24 de champ électromagnétique disposé de façon à émettre un champ électromagnétique selon une direction principale 9 sensiblement parallèle à la direction principale 13 d'émission. Une surface 124 de l'émetteur 24 est sensiblement perpendiculaire aux surfaces 120, 121, 122, 123. L'émetteur dit plan 24 permet d'avoir un champ électromagnétique parallèle à la direction principale 13 d'émission. L'émetteur dit plan 24 pouvant être par exemple une bobine ou un solénoïde, il est constitué d'au moins une spire dans laquelle peut circuler un courant. Et le flux de rayons X 12 peut traverser l'émetteur dit plan 24 au niveau de la spire. Autrement dit, l'émetteur dit plan 24 comprend au moins une spire traversée par la direction principale 13 d'émission du faisceau de rayons X 12. Le flux de rayons X 12 n'est pas obscurcit par l'émetteur dit plan 24 du fait qu'il le traverse à travers la ou les spires.

La disposition des émetteurs comme représentée sur la figure 2 permet d'avoir des champs électromagnétiques dont les directions principales sont selon trois axes différents perpendiculaires entre eux. Les émetteurs étant solidaires du tube générateur 11 du faisceau de rayons X 12, les champs électromagnétiques selon les trois axes permettent de déterminer la position des émetteurs par rapport au capteur plan 14 et donc de déterminer la position du tube générateur 11 du faisceau de rayons X 12 par rapport au capteur plan 14. On peut noter que les trois axes ne sont pas nécessairement perpendiculaires entre eux. Les directions 18 et 19 peuvent être sécantes et former un angle quelconque (entre elles et avec la direction principale 13 d'émission). La position relative du tube générateur 11 par rapport au capteur plan 14 peut également être déterminée.

Sur la figure 2, les émetteurs sont au nombre de trois (15, 16, 24, soit 4 parties d'émetteurs 20, 21, 22, 23 et un émetteur 24) et positionnés de manière à former un parallélépipède rectangle. Néanmoins, il est tout à fait envisageable d'avoir plus de trois émetteurs, chacun étant positionné sur une face d'un polyèdre dont le nombre de faces correspondrait au nombre de parties d'émetteurs et émetteurs utilisés. Un plus grand nombre d'émetteurs ajoute à la précision de la détermination du positionnement du capteur plan 14 par rapport au tube générateur 11. Néanmoins, ce plus grand nombre génère des coûts de production plus importants et une plus grande complexité de traitement des signaux. Trois émetteurs comme sur la figure 2 représentent un très bon compromis entre précision de la détermination du positionnement du capteur plan 14 par rapport au tube générateur 11 et complexité de traitement de signal.

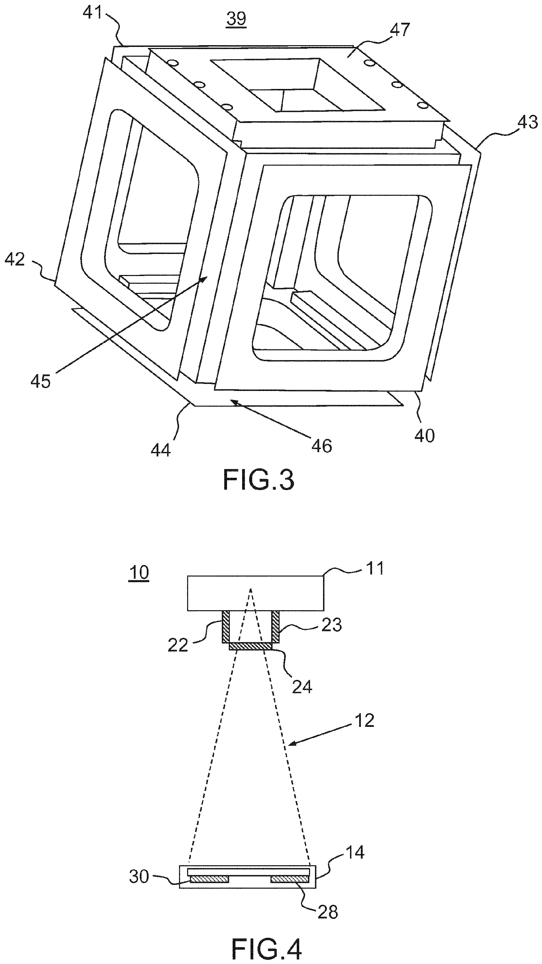

La figure 3 représente un exemple d'un support 39 des émetteurs de champ électromagnétique. Correspondant à la configuration de la figure 2, le support 39 dispose de faces 40, 41, 42, 43, 44 sensiblement perpendiculaires entre elles. La face 42 dispose d'une gorge 45 apte à recevoir la partie d'émetteur 22. De même, la face 44 dispose d'une gorge 46 apte à recevoir l'émetteur 24. Il en est de même pour chacune des faces. Le support 39 comprend un élément intermédiaire 47, sensiblement perpendiculaire aux faces 40, 41, 42, 43 et sensiblement parallèle à la face 44. L'élément intermédiaire 47 est un moyen de fixation permettant de rendre solidaire le support 39 (et donc les émetteurs 15, 16, 24) du tube générateur 11 du faisceau de rayons X 12.

Dans le cas d'une configuration avec plusieurs autres émetteurs, le support 39 a alors une autre forme géométrique à trois dimensions ayant des faces planes, chacune ayant une gorge disposée à loger un émetteur.

Grâce à la géométrie présentée aux figures 2 et 3, les surfaces deux à deux parallèles entre elles (120 et 121, 122 et 123) et le placement des émetteurs dans les gorges prévues à cet effet (par exemple une spire) font que les spires gauches et droites (des faces 42 et 43) et/ou avant et arrière (des faces 40 et 41) très symétriques permettent d'avoir un champ magnétique parfaitement centré sur le centre de la forme géométrique sans gêner le passage des rayons X. Il n'est pas nécessaire d'avoir plusieurs enroulements dans les gorges au niveau des faces latérales, l'enroulement inférieur, c'est-à-dire celui au niveau de la face 44 dans la gorge 46 étant suffisant pour la symétrie.

Autrement dit, chaque émetteur interrompu (15, 16) est interrompu en deux parties d'émetteur (20, 21 ; 22, 23) de champ électromagnétique configurées pour générer un champ électromagnétique parfaitement centré entre les deux faces que les parties d'émetteur constituent. Les deux parties d'émetteur ont chacune une surface, leurs deux surfaces sont parallèles entre elles.

Comme représenté sur la figure 1, l'ensemble de radiologie 10 comprend quatre capteurs 29, 30, 31, 32 de champ électromagnétique. Les quatre capteurs 29, 30, 31, 32 peuvent être intégrés dans le capteur plan 14. Les capteurs 29, 30, 31, 32 sont destinés à détecter les champs électromagnétiques émis par les émetteurs 15, 16, 24 et générer un signal électrique en fonction des champs électromagnétiques détectés. Il est à noter que l'ensemble de radiologie peut comprendre moins de quatre ou plus de quatre capteurs de champ électromagnétique.

Les capteurs 29, 30, 31, 32 sont intégrés dans le capteur plan 14. Ils sont installés de façon à ce qu'ils ne perturbent pas l'acquisition de l'image radiologique. Ils sont par exemple placés à l'arrière des éléments de détection de l'image radiologique par rapport à la face d'entrée des rayons X. Ils peuvent avoir une position quelconque sur le capteur plan 14. Dans ce cas, un correctif pour déterminer la position relative du tube générateur 11 par rapport au capteur plan 14 est nécessaire. Si, par contre, ils ont des positions parfaitement symétriques par rapport au centre du capteur plan, le centrage parfait par rapport au tube générateur 11 du faisceau de rayons X 12 est obtenu lorsque les capteurs 29, 30, 21, 32 ont un signal parfaitement équilibré.

Les capteurs 29, 30, 31, 32 de champ électromagnétique peuvent être par exemple des bobines, des magnétomètres, des magnétorésistances, des magnétorésistances anisotropiques, des magnéto-transistors, des magnéto-diodes, des flux gâtes ou des senseurs à effet Hall.

La figure 4 représente une vue en coupe de l'ensemble de radiologie 10 selon l'invention. Chacun des capteurs 29, 30, 31, 32 de champ électromagnétique peut comprendre un circuit électronique d'amplification et de filtrage (non représentés sur la figure) destiné à traiter le signal électrique généré par chacun des capteurs 29, 30, 31, 32.

Le moyen de traitement 17 comprend en outre un calculateur apte à calculer la position relative du capteur plan 14 par rapport au tube générateur 11.

Chaque capteur 29, 30, 31, 32 détecte un champ électromagnétique et génère un signal électrique fonction de l'amplitude du champ électromagnétique détecté. Le signal électrique généré est traité par le circuit électronique d'amplification et de filtrage.

Selon le type de capteur utilisé, à chaque instant, chaque capteur 29, 30, 31, 32 peut générer une seule ou plusieurs informations. Si le capteur est mono-axe, il génère une seule information. Si le capteur est multi-axes, il génère plusieurs informations. L'utilisation de capteurs multi-axes permet de connaître l'amplitude du champ électromagnétique ainsi que son orientation.

Les signaux détectés sont numérisés et transmis au calculateur qui traite les informations afin de déterminer la position relative du capteur plan 14 par rapport au tube générateur 11 du faisceau de rayons X. Les informations issues des capteurs 29, 30, 31, 32 sont transmises sous forme numérique. Elles peuvent transiter soit par liaison filaire soit par liaison sans fil.

Pour une position donnée du capteur plan 14, la position du capteur plan 14 dans l'espace est déterminée à partir d'un jeu d'informations, notamment les informations générées par chacun des capteurs 29, 30, 31, 32 de champ électromagnétique quand l'émetteur 15 est alimenté, les informations générées par chacun des capteurs 29, 30, 31, 32 de champ électromagnétique quand l'émetteur 16 est alimenté et enfin les informations générées par chacun des capteurs 29, 30, 31, 32 de champ électromagnétique quand l'émetteur 24 est alimenté.

Dans notre configuration, si les capteurs sont des capteurs mono-axe, on dispose pour une position donnée du capteur plan 14 de douze informations. Si les capteurs sont des capteurs multi-axes, on dispose alors de trente-six informations.

Des calculs utilisant l'ensemble de ces informations permettent de connaître sans ambiguïté la position dans l'espace du capteur plan 14.

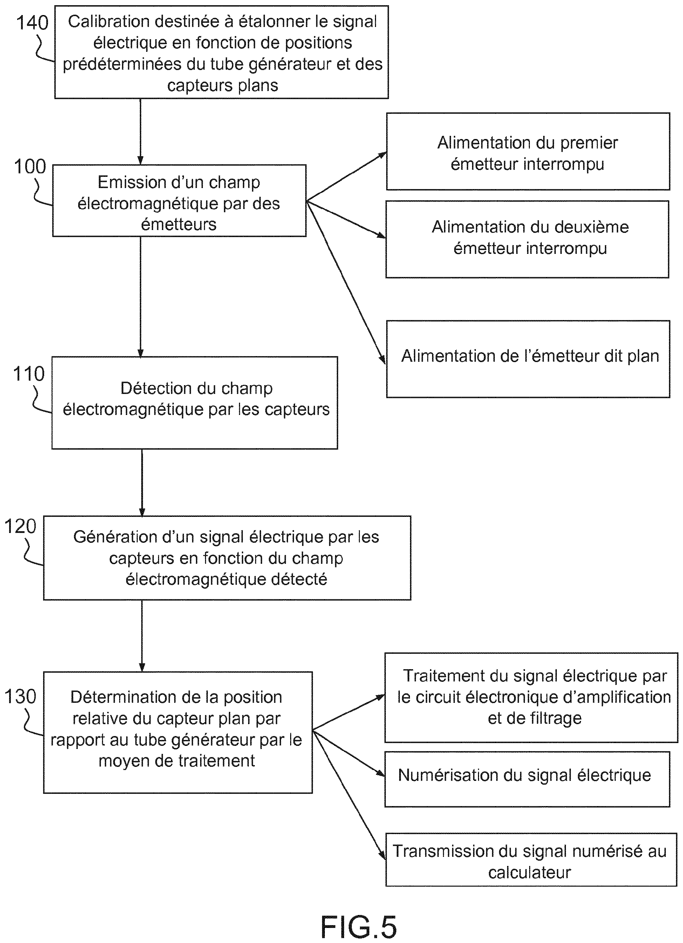

La figure 5 représente schématiquement les étapes d'un procédé d'alignement selon l'invention. Le procédé d'alignement d'un ensemble de radiologie 10 selon l'invention comporte les étapes suivantes :
- Emission par un émetteur 15, 16 ou 24 d'un champ électromagnétique (étape 100),
- Détection du champ électromagnétique par les capteurs 29, 30, 31, 32 (étape 110),
- Génération d'un signal électrique par les capteurs 29, 30, 31, 32 en fonction du champ électromagnétique détecté (étape 120),
- Détermination de la position relative du capteur plan 14 par rapport au tube générateur 11 par le moyen de traitement 17 en fonction du signal électrique (étape 130).

Dans le cas d'un ensemble de radiologie 10 comprenant plusieurs émetteurs 15, 16, 24, le moyen de traitement 17 peut comprendre des moyens pour distinguer les signaux électriques générés. Le procédé comporte alors les étapes suivantes :
- Emission par le deuxième émetteur du champ électromagnétique (étape 100),
- Détection du champ électromagnétique par les capteurs (étape 110),
- Génération d'un signal électrique par les capteurs 29, 30, 31, 32 en fonction du champ électromagnétique détecté (étape 120), distinct du signal électrique généré par les capteurs 29, 30, 31, 32 en fonction du champ électromagnétique émis par le premier émetteur,
- Détermination de la position relative du capteur plan 14 par rapport au tube générateur 11 par le moyen de traitement 17 en fonction du signal électrique (étape 130).

L'étape 130 de détermination de la position relative du capteur plan par rapport au tube générateur comporte les étapes suivantes :
- Traitement du signal électrique par le circuit électronique d'amplification et de filtrage (étape 131),
- Numérisation du signal électrique (étape 132),
- Transmission du signal numérisé au calculateur (étape 133).

Le procédé d'alignement selon l'invention peut comporter au préalable une étape 140 de calibration destinée à étalonner le signal électrique en fonction de positions prédéterminées du tube générateur 11 et du capteur plan 14. Lors de cette étape, la position relative des différents éléments (capteur plan 14, tube générateur 11) est enregistrée puis utilisée pour déterminer des termes correctifs qui seront pris en compte dans l'étape 130 de détermination de la position relative du capteur plan 14 par rapport au tube générateur 11.

Le procédé selon l'invention permet donc d'éviter de sur-irradier le patient, placé entre le tube générateur 11 et le capteur plan 14, avec des rayons X arrivant en dehors du capteur plan 14.

L'étape 100 d'émission par le au moins un émetteur 15, 16 du au moins un champ électromagnétique comporte les étapes suivantes :
- Alimentation du premier émetteur interrompu 15 de façon à ce qu'il émette un premier champ électromagnétique dont la direction principale 18 est sensiblement perpendiculaire à la direction principale 13 d'émission,
- Alimentation du deuxième émetteur interrompu 16 de façon à ce qu'il émette un deuxième champ électromagnétique dont la direction principale 19 est sensiblement perpendiculaire à la direction principale 13 d'émission et sécante à la direction principale 18 du premier champ électromagnétique.

L'étape 100 d'émission par l'émetteur 15, 16, 24 du n champ électromagnétique peut comporter l'étape suivante :
- Alimentation de l'émetteur dit plan 24 de façon à ce qu'il émette un troisième champ électromagnétique dont la direction principale 9 est sensiblement parallèle à la direction principale 13 d'émission.

Enfin, l'alimentation des émetteurs 15, 16, 24 par les signaux électriques se fait à des instants différents ou simultanément à des fréquences différentes ou simultanément en décalage de phase de façon à différencier les champs électromagnétiques émis.

Autrement dit, l'alimentation du premier émetteur interrompu 15 et l'alimentation du deuxième émetteur interrompu 16 peuvent se faire à des instants différents ou simultanément à une fréquence différente ou en décalage de phase. Le fait d'alimenter les émetteurs interrompus à des instants différents ou simultanément à une fréquence différente ou en décalage de phase est un moyen pour distinguer les signaux électriques générés.

De même, l'alimentation de l'émetteur dit plan 24 et l'alimentation d premier émetteur interrompu 15 et du deuxième émetteur interrompu 16 peuvent se faire à des instants différents ou simultanément à des fréquences différentes ou en décalage de phase.

Généralement, l'alimentation des émetteurs se fait par des signaux alternatifs afin de s'affranchir des signaux continus liés à la présence d'aimants et au champ magnétique terrestre. La fréquence typique d'alimentation est comprise entre 100 Hz et 10kHz.

L'alimentation des émetteurs se fait à des instants différents pour que les capteurs puissent séparer aisément les champs électromagnétiques détectés. Dans le cas où l'alimentation des émetteurs se fait simultanément, les fréquences sont différentes, et le choix des fréquences est tel que les signaux peuvent être facilement séparés en sortie des capteurs de champ électromagnétique.

Lorsque le procédé d'alignement est activé par un opérateur, les émetteurs 15, 16, 24 de champ électromagnétique solidaires du tube générateur 11 du faisceau de rayons X 12 sont activés via une liaison numérique. La fréquence du champ électromagnétique de chaque émetteur est programmable. Chaque émetteur peut donc émettre à une fréquence propre distincte des autres. Les capteurs 29, 30, 31, 32 de champ électromagnétique solidaires du capteur plan 14 reçoivent le signal électromagnétique des émetteurs 15, 16, 24. Le niveau et la fréquence du signal détecté par chacun des capteurs sont transmis par une liaison numérique au moyen de traitement 17. Le moyen de traitement 17 traite les données et fournit à l'opérateur les informations nécessaires pour qu'il aligne manuellement le tube générateur 11 en face du capteur plan 14. L'opérateur déplace le tube générateur 11 ou le capteur plan 14 afin d'optimiser l'alignement de l'un par rapport à l'autre. Les informations permettant l'alignement sont fournies par exemple sur un écran d'affichage intégré sur le tube générateur 11 ou sur un autre élément relié à l'ensemble de radiologie. Un signal sonore, dont la fréquence est modulée en fonction de la distance rapport à la position optimale, peut également indiquer à l'utilisateur la précision de l'alignement.

## Revendications

1. Ensemble de radiologie (10) comprenant :
• un tube générateur (11) d'un faisceau de rayons X (12) centré autour d'une direction principale (13) d'émission,
• un capteur plan (14) sensiblement perpendiculaire à la direction principale (13) d'émission, destiné à recevoir les rayons X (12),
**caractérisé en ce qu'**il comprend :
• un premier émetteur interrompu (15, 16) en deux parties d'émetteur (20, 21 ; 22, 23) de champ électromagnétique, disposé de façon à émettre un premier champ électromagnétique selon une direction principale (18, 19) sensiblement perpendiculaire à la direction principale (13) d'émission, chacune des deux parties d'émetteur (20, 21 ; 22, 23) de l'émetteur interrompu (15, 16) étant positionnée de part et d'autre du faisceau de rayons X (12),
• un deuxième émetteur interrompu (16) en deux parties d'émetteur (22, 23) de champ électromagnétique, disposé de façon à émettre un deuxième champ électromagnétique selon une direction principale (19) sensiblement perpendiculaire à la direction principale (13) d'émission et sécante à la direction principale (18) du premier champ électromagnétique, chacune des deux parties (22, 23) de l'émetteur interrompu (16) étant positionnée de part et d'autre du faisceau de rayons X (12),
• un émetteur dit plan (24) de champ électromagnétique, l'émetteur dit plan (24) étant une bobine composée de spires, l'émetteur dit plan (24) étant disposé de façon à émettre un troisième champ électromagnétique selon une direction principale (9) sensiblement parallèle à la direction principale (13) d'émission, les spires étant traversées par la direction principale (13) d'émission,
• des capteurs (29, 30, 31, 32) de champ électromagnétique solidaires du capteur plan (14), aptes à détecter les premier, deuxième et troisième champs électromagnétiques émis alternativement selon leur direction principale par le premier émetteur (15, 16), le deuxième émetteur (15, 16) et l'émetteur dit plan (24) et générer un premier, deuxième, troisième signal électrique en fonction des champs électromagnétiques détectés,
• un moyen de traitement (17) du premier, deuxième, troisième signal électrique destiné à déterminer la position relative du capteur plan (14) par rapport au tube générateur (11) en fonction du premier, deuxième, troisième signal électrique.

2. Ensemble de radiologie (10) selon la revendication 1, **caractérisé en ce que** le moyen de traitement (17) comprend des moyens pour distinguer les signaux électriques générés.

3. Ensemble de radiologie (10) selon l'une des revendications 1 ou 2, **caractérisé en ce que** chacune des deux parties d'émetteur (20, 21 ; 22, 23) de l'émetteur interrompu (15, 16) comprend au moins une spire et **en ce que** la direction principale (13) d'émission du faisceau de rayons X (12) est positionnée entre les au moins une spire de l'émetteur interrompu (15, 16).

4. Ensemble de radiologie (10) selon l'une des revendications 1 à 3, **caractérisé en ce que** l'émetteur dit plan (24) comprend au moins une spire traversée par la direction principale (13) d'émission du faisceau de rayons X (12).

5. Procédé d'alignement d'un ensemble de radiologie (10) selon l'une des revendications précédentes, **caractérisé en ce qu'**il comporte les étapes suivantes :
• Emission par le premier émetteur du premier champ électromagnétique (étape 100) selon la direction principale (18, 19) sensiblement perpendiculaire à la direction principale (13) d'émission,
• Emission par le deuxième émetteur du deuxième champ électromagnétique selon la direction principale (18, 19) sensiblement perpendiculaire à la direction principale (13) d'émission,
• Emission par l'émetteur dit plan d'un troisième champ électromagnétique selon une direction principale (9) sensiblement parallèle à la direction principale (13) d'émission,
• Détection par les capteurs des champs électromagnétiques émis alternativement selon leur direction principale par le premier émetteur (15, 16), le deuxième émetteur (15, 16) et l'émetteur dit plan (24) (étape 110),
• Génération des premier, deuxième, troisième signaux électriques par les capteurs (29, 30, 31, 32) en fonction des premier, deuxième, troisième champs électromagnétiques détectés (étape 120),
• Détermination de la position relative du capteur plan (14) par rapport au tube générateur (11) par le moyen de traitement (17) en fonction du premier, deuxième, troisième signal électrique (étape 130).

6. Procédé d'alignement selon la revendication 5, **caractérisé en ce qu'**il comporte au préalable une étape (140) de calibration destinée à étalonner le signal électrique en fonction de positions prédéterminées du tube générateur (11) et du capteur plan (14).

7. Procédé d'alignement selon l'une des revendications 5 ou 6, **caractérisé en ce que** l'émission par les émetteurs des champs électromagnétiques comporte une étape d'alimentation des émetteurs (15, 16, 24), et **en ce que** l'alimentation des émetteurs se fait à des instants différents ou simultanément à des fréquences différentes ou simultanément en décalage de phase de façon à différencier les champs électromagnétiques émis.

## Patentansprüche

1. Radiologische Anordnung (10), Folgendes umfassend:
• eine Röhre (11) zum Erzeugen eines Röntgenstrahls (12), welche um eine Haupt-Emissionsrichtung (13) zentriert ist,
• einen ebenen Sensor (14), welcher im Wesentlichen zur Haupt-Emissionsrichtung (13) rechtwinklig ist, dazu bestimmt, die Röntgenstrahlen (12) zu empfangen,
**dadurch gekennzeichnet, dass** er Folgendes umfasst:
• einen ersten Sender (15, 16), der in zwei Senderabschnitten (20, 21; 22, 23) eines elektromagnetischen Feldes unterbrochen ist, dergestalt angeordnet, dass er ein erstes elektromagnetisches Feld entlang einer Hauptrichtung (18, 19) aussendet, welche im Wesentlichen rechtwinklig zur Hauptemissionsrichtung (13) ist, wobei jeder der beiden Senderabschnitte (20, 21, 22, 23) des unterbrochenen Senders (15, 16) auf beiden Seiten des Röntgenstrahls (12) positioniert ist,
• einen zweiten Sender (16), der in zwei Senderabschnitten (22, 23) eines elektromagnetischen Feldes unterbrochen ist, dergestalt angeordnet, dass er ein zweites elektromagnetisches Feld entlang einer Hauptrichtung (19) sendet, welche im Wesentlichen rechtwinklig zur Haupt-Emissionsrichtung (13) ist und eine Sekante der Hauptrichtung (18) des ersten elektromagnetischen Feldes ist, wobei jeder der beiden Senderabschnitte (22, 23) des unterbrochenen Senders (16) auf beiden Seiten des Röntgenstrahls (12) positioniert ist
• einen eben genannten Sender (24) eines elektromagnetischen Feldes, wobei der eben genannte Sender (24) eine aus Windungen bestehende Spule ist, wobei der eben genannte Sender (24) dergestalt angeordnet ist, dass er ein drittes elektromagnetisches Feld entlang einer Hauptrichtung (9) sendet, welche im Wesentlichen parallel zur Haupt-Emissionsrichtung (13) ist, wobei die Windungen durch die Haupt-Emissionsrichtung (13) durchquert werden,
• Sensoren (29, 30, 31, 32) eines elektromagnetischen Feldes, welche mit dem ebenen Sensor (14) fest verbunden und in der Lage sind, das erste, das zweite und das dritte elektromagnetische Feld zu erkennen, welche abwechselnd entlang ihrer Hauptrichtung durch den ersten Sender (15, 16), den zweiten Sender (15, 16) und den eben genannten Sender (24) ausgesendet werden, und ein erstes, ein zweites und ein drittes elektrisches Signal angesichts der erkannten elektromagnetischen Felder zu erzeugen,
• ein Verarbeitungsmittel (17) des ersten, des zweiten und des dritten elektrischen Signals, welches dazu bestimmt ist, die relative Position des ebenen Sensors (14) in Bezug auf die Erzeugungsröhre (11) angesichts des ersten, des zweiten und des dritten elektrischen Signals zu bestimmen.

2. Radiologische Anordnung (10) nach Anspruch 1, **dadurch gekennzeichnet, dass** das Verarbeitungsmittel (17) Mittel zum Unterscheiden der erzeugten elektrischen Signale umfasst.

3. Radiologische Anordnung (10) nach einem der Ansprüche 1 oder 2, **dadurch gekennzeichnet, dass** jeder der zwei Senderabschnitte (20, 21; 22, 23) des unterbrochenen Senders (15, 16) mindestens eine Windung umfasst, und dass die Haupt-Emissionsrichtung (13) des Röntgenstrahls (12) zwischen den mindestens einer Windung des unterbrochenen Senders (15, 16) positioniert ist.

4. Radiologische Anordnung (10) nach einem der Ansprüche 1 bis 3, **dadurch gekennzeichnet, dass** der eben genannte Sender (24) mindestens eine durch die Haupt-Emissionsrichtung (13) des Röntgenstrahls (12) durchquerte Windung umfasst.

5. Verfahren zur Ausfluchtung einer radiologischen Anordnung (10) nach einem der vorhergehenden Ansprüche, **dadurch gekennzeichnet, dass** es folgende Schritte umfasst:
• Aussenden, durch den ersten Sender, des ersten elektromagnetischen Feldes (Schritt 100) entlang der Hauptrichtung (18, 19), welche im Wesentlichen rechtwinklig zur Haupt-Emissionsrichtung (13) ist,
• Aussenden, durch den zweiten Sender, des zweiten elektromagnetischen Feldes entlang der Hauptrichtung (18, 19), welche im Wesentlichen rechtwinklig zur Haupt-Emissionsrichtung (13) ist,
• Aussenden, durch den eben genannten Sender, eines Dritten elektromagnetischen Feldes entlang einer Hauptrichtung (9), welche im Wesentlichen parallel zur Haupt-Emissionsrichtung (13) ist,
• Erkennen, durch die Sensoren, der abwechselnd entlang ihrer Hauptrichtung durch den ersten Sender (15, 16), den zweiten Sender (15, 16) und den eben genannten Sender (24) (Schritt 110) ausgesendeten elektromagnetischhen Felder,
• Erzeugen des ersten, des zweiten und des dritten elektrischen Signals durch die Sensoren (29, 30, 31, 32) angesichts des ersten, des zweiten und des dritten erkannten elektromagnetischen Feldes (Schritt 120),
• Bestimmen der relativen Position des ebenen Sensors (14) in Bezug auf die Erzeugungsröhre (11) durch das Verarbeitungsmittel (17) angesichts des ersten, des zweiten und des dritten elektrischen Signals (Schritt 130).

6. Verfahren zur Ausfluchtung nach Anspruch 5, **dadurch gekennzeichnet, dass** es zuvor einen Kalibrierungsschritt (140) aufweist, welcher dazu bestimmt ist, das elektrische Signal angesichts von vorbestimmten Positionen der Erzeugungsröhre (11) und des ebenen Sensors (14) zu eichen.

7. Verfahren zur Ausfluchtung nach einem der Ansprüche 5 oder 6, **dadurch gekennzeichnet, dass** die Aussendung durch die Sender der elektromagnetischen Felder einen Schritt der Speisung der Sender (15, 16, 24) aufweist, und dadurch, dass die Speisung der Sender zu unterschiedlichen Zeitpunkten oder gleichzeitig mit unterschiedlichen Frequenzen oder gleichzeitig mit Phasenverschiebung dergestalt erfolgt, dass die ausgesendeten elektromagnetischen Felder differenziert werden.

## Claims

1. A radiology assembly (10) comprising:
• a tube (11) for generating a beam of x-rays (12) that is centered on a main emission direction (13),
• a planar sensor (14), substantially perpendicular to the main emission direction (13), that is intended to receive the x-rays (12),
**characterized in that** it comprises:
• a first emitter (15, 16) that is divided into two electromagnetic-field-emitting portions (20, 21; 22, 23), placed so as to emit a first electromagnetic field in a main direction (18, 19) that is substantially perpendicular to the main emission direction (13), each of the two emitting portions (20, 21; 22, 23) of the divided emitter (15, 16) being positioned on both sides of the beam of x-rays (12),
• a second emitter (16) that is divided into two electromagnetic-field-emitting portions (22, 23), placed so as to emit a second electromagnetic field in a main direction (19) that is substantially perpendicular to the main emission direction (13) and that is secant to the main direction (18) of the first electromagnetic field, each of the two portions (22, 23) of the divided emitter (16) being positioned on both sides of the beam of x-rays (12),
• a so-called planar electromagnetic-field emitter (24), the so-called planar emitter (24) being a coil composed of windings, the so-called planar emitter (24) being placed so as to emit a third electromagnetic field in a main direction (9) that is substantially parallel to the main emission direction (13), the windings being passed through by the main emission direction (13),
• electromagnetic-field sensors (29, 30, 31, 32) that are securely fastened to the planar sensor (14), able to detect the first, second and third electromagnetic field alternatively emitted in their main direction by the first emitter (15, 16), the second emitter (15, 16) and the so-called planar emitter (24) and to generate a first, second, third electrical signal depending on the detected electromagnetic fields,
• a means (17) for processing the first, second and third electrical signal, which is intended to determine the relative position of the planar sensor (14) with respect to the generation tube (11) depending on the first, second and third electrical signal.

2. The radiology assembly (10) according to claim 1, **characterized in that** the processing means (17) comprises means for distinguishing the generated electrical signals.

3. The radiology assembly (10) according to one of claims 1 or 2, **characterized in that** each of the two emitting portions (20, 21; 22, 23) of the divided emitter (15, 16) comprises a least one winding and **in that** the main emission direction (13) of the beam of x-rays (12) is positioned between the at least one winding of the divided emitter (15, 16).

4. The radiology assembly (10) according to one of claims 1 to 3, **characterized in that** the so-called planar emitter (24) comprises at least one winding that is passed through by the main emission direction (13) of the beam of x-rays (12).

5. A method for aligning a radiology assembly (10) according to one of the preceding claims, **characterized in that** it includes the following steps:
• emission by the first emitter of the first electromagnetic field (step 100) in the main direction (18, 19) that is substantially perpendicular to the main emission direction (13),
• emission by the second emitter of the second electromagnetic field in the main direction (18, 19) that is substantially perpendicular to the main emission direction (13),
• emission by the so-called plane emitter of a third electromagnetic field in a main direction (9) that is substantially parallel to the main emission direction (13),
• detection by the sensors of the electromagnetic fields alternatively emitted in their main direction by the first emitter (15, 16), the second emitter (15, 16) and the so-called plane emitter (24) (step 110);
• generation of the first, second and third electrical signals by the sensors (29, 30, 31, 32) depending on the detected first, second, third electromagnetic fields (step 120),
• determination of the relative position of the planar sensor (14) with respect to the generation tube (11) by the processing means (17) depending on the first, second and third electrical signal (step 130).

6. The aligning method according to claim 5, **characterized in that** it includes, beforehand, a calibration step (140) intended to calibrate the electrical signal as a function of preset positions of the generation tube (11) and of the planar sensor (14).

7. The aligning method according to one of claims 5 or 6, **characterized in that** the emission by the emitters of the electromagnetic fields includes a step of supplying the emitters (15, 16, 24) with power, and **in that** the emitters are supplied with power at different times or simultaneously at different frequencies or simultaneously with a phase offset so as to differentiate the electromagnetic fields emitted.
